# EUROPEAN PATENT APPLICATION

(11) **EP 4 850 742 A1**
(43) Date of publication of application: **09.12.2026**
(21) Application number: 25757799.9
(22) Date of filing: 18.02.2025
(51) Int. Cl.: A61M 39/10

(54) **CONNECTION STRUCTURE**

(30) Priority: 21.02.2024 JP 2024024831
(71) Applicant: Terumo Kabushiki Kaisha, Tokyo 151-0072 (JP)
(72) Inventor: KURIYAMA, Tasuku, Nakakoma-gun, Yamanashi 409-3853 (JP); UEDA, Yasuhiro, Nakakoma-gun, Yamanashi 409-3853 (JP); AKIYAMA, Kazuya, Nakakoma-gun, Yamanashi 409-3853 (JP)
(74) Representative: KIPA AB
(86) International application number: PCT/JP2025/005435
(87) International publication number: WO 2025/178030

(57) **Abstract**

A connection structure including a female connector and a male connector, in which the female connector includes a connector body having a connection port and an elastic valve body, the elastic valve body includes a valve body and a fixing portion, the male connector includes a flow passage tubular member having an opening, an elastic opening/closing portion, a biasing portion, and a housing, the male connector changes from a first form in which the opening is closed to a second form in which the opening is opened by a connecting operation, the housing has a housing restriction portion, the valve body has a slit and a protruding portion provided on a bottom surface, the elastic opening/closing portion has an inserted portion and a slit, and the connector body has a rib against which the protruding portion is pressed as the valve body is pressed toward a deep side of the connection port by the inserted portion by the connecting operation.

## Description

### Technical Field

The present disclosure relates to a connection structure.

### Background Art

A connection structure is known, the connection structure including: a female connector; and a male connector connectable to the female connector, in which the male connector includes a flow passage tubular member that defines an axial direction and has an opening at a portion on an axially distal side, an elastic opening/closing portion, and a biasing portion capable of biasing the elastic opening/closing portion toward the axially distal side, and the male connector changes from a first form in which the opening of the flow passage tubular member is closed by the elastic opening/closing portion to a second form in which the opening of the flow passage tubular member is opened by a connecting operation where an elastic valve body of the female connector is pressed against a distal surface of the elastic opening/closing portion against a biasing force of the biasing portion to cause the elastic opening/closing portion to move toward an axially proximal side relative to the flow passage tubular member, such that the portion on the axially distal side of the flow passage tubular member protrudes from the elastic opening/closing portion toward the axially distal side (for example, see Patent Literature 1).

### Citation List

### Patent Literature

Patent Literature 1: WO 2022/149339 A1

### Summary of Invention

### Technical Problem

An object of the present disclosure is to provide a connection structure capable of suppressing fluid leakage due to favorable tight contact between the elastic opening/closing portion of the male connector and the elastic valve body of the female connector at the time of connection.

### Solution to Problem

An aspect of the present disclosure is as follows.
[1] A connection structure including: a female connector; and a male connector connectable to the female connector,
   in which the female connector includes a connector body having a connection port and an elastic valve body,
   the elastic valve body includes a valve body that closes the connection port and a fixing portion that extends from an outer peripheral surface of the valve body and is fixed to the connector body,
   the male connector includes a flow passage tubular member that defines an axial direction and has an opening at a portion on an axially distal side, an elastic opening/closing portion, a biasing portion capable of biasing the elastic opening/closing portion toward the axially distal side, and a housing continuous with the flow passage tubular member,
   the male connector changes from a first form in which the opening of the flow passage tubular member is closed by the elastic opening/closing portion to a second form in which the opening of the flow passage tubular member is opened by a connecting operation where the elastic valve body is pressed against a distal surface of the elastic opening/closing portion against a biasing force of the biasing portion to cause the elastic opening/closing portion to move toward an axially proximal side relative to the flow passage tubular member, such that the portion on the axially distal side of the flow passage tubular member protrudes from the elastic opening/closing portion toward the axially distal side,
   the housing has a housing restriction portion that restricts movement of the female connector toward the axially distal side relative to the elastic opening/closing portion in accordance with a change from the first form to the second form,
   the valve body has a slit penetrating the valve body and a protruding portion provided on a bottom surface so as to surround the slit,
   the elastic opening/closing portion has an inserted portion inserted into the connection port by the connecting operation and a slit penetrating the elastic opening/closing portion through the inserted portion, and
   the connector body has a rib against which the protruding portion is pressed as the valve body is pressed toward a deep side of the connection port by the inserted portion by the connecting operation.
[2] The connection structure according to [1], in which the inserted portion has an outer diameter equal to or less than an inner diameter of the connection port of the connector body.
[3] The connection structure according to [1] or [2], in which the flow passage tubular member defines a radial direction,
   the male connector includes a movable body having a holding opening in which the elastic opening/closing portion is disposed and held and a movable body restriction portion that restricts the movement of the female connector toward the axially distal side relative to the elastic opening/closing portion by moving radially inward by a reaction force from the housing generated in accordance with the connecting operation, and
   the inserted portion of the elastic opening/closing portion protrudes from the holding opening toward the axially distal side.
[4]
   The connection structure according to any one of [1] to [3], in which the protruding portion is provided in an annular shape so as to surround the slit of the valve body.
[5] The connection structure according to any one of [1] to [4], in which the rib is provided in an annular shape so as to surround the connection port.
[6] The connection structure according to any one of [1] to [5], in which each of the male connector and the female connector is configured as a medical device.

### Advantageous Effects of Invention

According to the present disclosure, it is possible to provide a connection structure capable of suppressing fluid leakage due to favorable tight contact between an elastic opening/closing portion of a male connector and an elastic valve body of a female connector at the time of connection.

### Brief Description of Drawings

Fig. 1 is a cross-sectional view illustrating a state before connection in a connection structure according to an embodiment of the present disclosure.
Fig. 2 is a cross-sectional view illustrating the state of Fig. 1 at an angle different by 90°.
Fig. 3 is a cross-sectional view illustrating a state when a connecting operation is started from the state of Fig. 1.
Fig. 4 is a cross-sectional view illustrating a state when the connecting operation is further performed and completed from the state of Fig. 3.
Fig. 5 is a cross-sectional view illustrating the state of Fig. 4 at an angle different by 90°.

### Description of Embodiments

An embodiment of the present disclosure will be described in detail below with reference to the drawings.

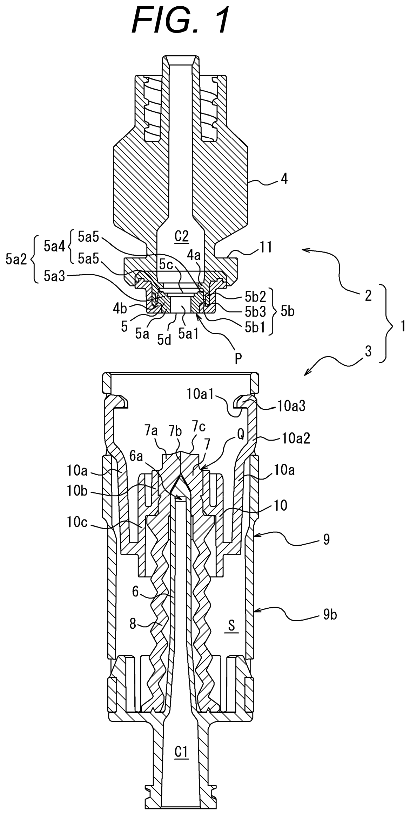

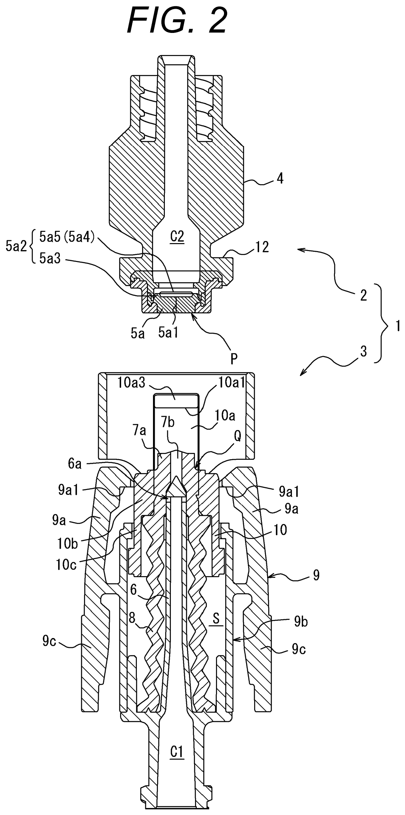

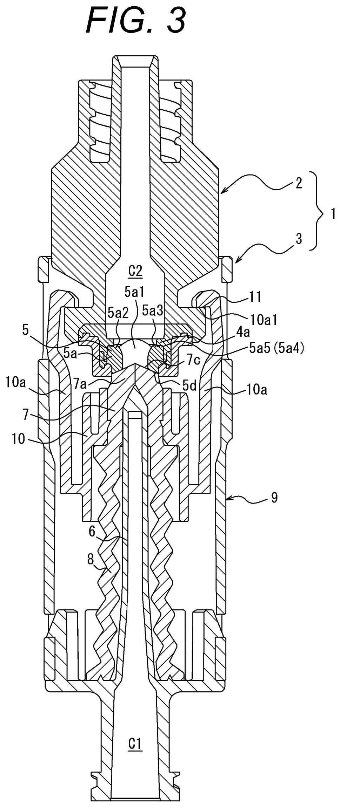

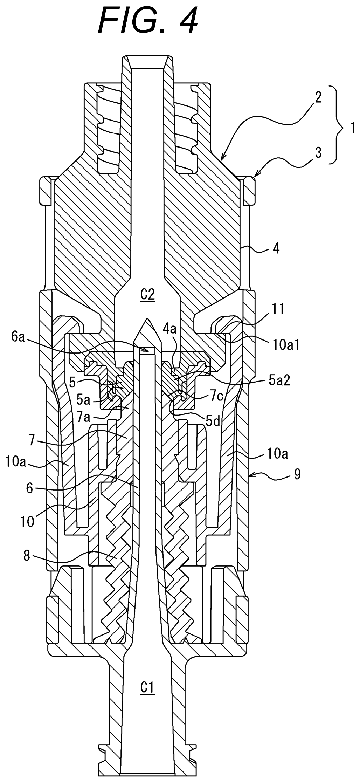

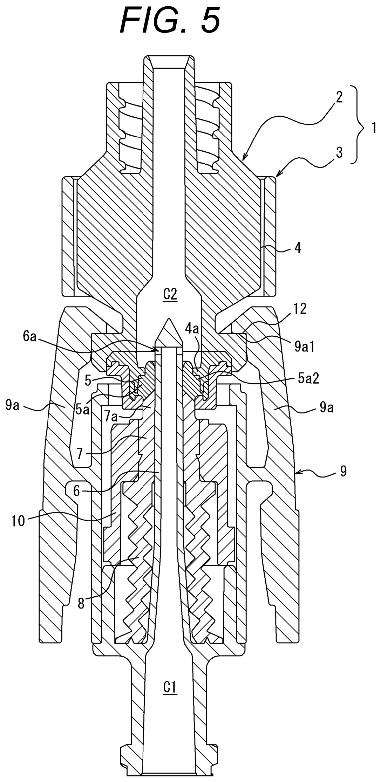

As illustrated in Figs. 1 to 5, a connection structure 1 according to the embodiment of the present disclosure includes a female connector 2 and a male connector 3 connectable to the female connector 2. The female connector 2 includes a connector body 4 having a connection port P and an elastic valve body 5. The elastic valve body 5 includes a valve body 5a that closes the connection port P and a fixing portion 5b that extends from an outer peripheral surface of the valve body 5a and is fixed to the connector body 4. The male connector 3 includes a flow passage tubular member 6 that defines an axial direction and has an opening 6a at a portion on an axially distal side, an elastic opening/closing portion 7, a biasing portion 8 capable of biasing the elastic opening/closing portion 7 toward the axially distal side, and a housing 9 continuous with the flow passage tubular member 6. The male connector 3 changes a form from a first form (see Figs. 1 and 2) in which the opening 6a of the flow passage tubular member 6 is closed by the elastic opening/closing portion 7 to a second form (see Figs. 4 and 5) in which the opening 6a of the flow passage tubular member 6 is opened by a connecting operation where the elastic valve body 5 is pressed against a distal surface 7c of the elastic opening/closing portion 7 against a biasing force of the biasing portion 8 to cause the elastic opening/closing portion 7 to move toward an axially proximal side relative to the flow passage tubular member 6, such that the portion on the axially distal side of the flow passage tubular member 6 protrudes from the elastic opening/closing portion 7 toward the axially distal side. The housing 9 has a housing restriction portion 9a that restricts the movement of the female connector 2 toward the axially distal side relative to the elastic opening/closing portion 7 in accordance with the change from the first form to the second form. The valve body 5a has a slit 5a1 penetrating the valve body 5a and a protruding portion 5a2 provided on a bottom surface 5c so as to surround the slit 5a1. The elastic opening/closing portion 7 has an inserted portion 7a inserted into the connection port P by the connecting operation and a slit 7b penetrating the elastic opening/closing portion 7 through the inserted portion 7a. The connector body 4 has a rib 4a against which the protruding portion 5a2 is pressed as the valve body 5a is pressed toward a deep side of the connection port P by the inserted portion 7a through the connecting operation.

According to the above configuration, the first form in which the flow passage (male connector flow passage C1) inside the flow passage tubular member 6 of the male connector 3 is closed by the elastic opening/closing portion 7 and the flow passage (female connector flow passage C2) inside the connector body 4 of the female connector 2 is closed by the elastic valve body 5 is changed to the second form as the elastic valve body 5 is pressed against the elastic opening/closing portion 7 and moved through the connecting operation, and the male connector flow passage C1 and the female connector flow passage C2 can communicate with each other via the opening 6a of the flow passage tubular member 6 to cause a fluid to flow. At the time of the connecting operation, since the inserted portion 7a of the elastic opening/closing portion 7 is inserted into the connection port P of the female connector 2 by the biasing force of the biasing portion 8, the top surface 5d of the elastic valve body 5 fixed to the connector body 4 via the fixing portion 5b and located inside the connection port P can be firmly pressed by the inserted portion 7a. Furthermore, at this time, the elastic valve body 5 pressed by the inserted portion 7a can be supported in the opposite direction by the rib 4a provided in the connector body 4 of the female connector 2 via the protruding portion 5a2 provided on the bottom surface 5c of the elastic valve body 5. Therefore, it is possible to ensure favorable tight contact between the top surface 5d of the elastic valve body 5 and the distal surface 7c of the elastic opening/closing portion 7 at the time of the connecting operation. In this state, since the flow passage tubular member 6 can pass through the slit 7b of the elastic opening/closing portion 7 and the slit 5a1 of the elastic valve body 5, it is possible to suppress the leakage of the fluid during the connecting operation from the first form to the second form. Furthermore, when the second form is reached by the connecting operation, the connection state is favorably maintained by the housing restriction portion 9a. Therefore, it is possible to achieve the connection structure 1 capable of suppressing fluid leakage due to favorable tight contact between the elastic opening/closing portion 7 of the male connector 3 and the elastic valve body 5 of the female connector 2 at the time of connection.

The inserted portion 7a has an outer diameter equal to or less than an inner diameter of the connection port P of the connector body 4. According to the above configuration, since the inserted portion 7a is easily inserted into the connection port P of the female connector 2 at the time of the connecting operation, the tight contact between the elastic valve body 5 and the elastic opening/closing portion 7 can be improved.

The flow passage tubular member 6 defines a radial direction, the male connector 3 includes a movable body 10 having a holding opening Q in which the elastic opening/closing portion 7 is disposed and held and a movable body restriction portion 10a that restricts the movement of the female connector 2 toward the axially distal side relative to the elastic opening/closing portion 7 by moving radially inward by the reaction force from the housing 9 generated in accordance with the connecting operation, and the inserted portion 7a of the elastic opening/closing portion 7 protrudes from the holding opening Q toward the axially distal side. According to the above configuration, the state where the elastic valve body 5 is pressed against the distal surface 7c of the elastic opening/closing portion 7 at the time of the connecting operation can be favorably maintained by the movable body restriction portion 10a.

In the present embodiment, the protruding portion 5a2 does not contact the rib 4a in the first form. However, the protruding portion 5a2 may be in contact with the rib 4a in the first form.

The protruding portion 5a2 is provided in an annular shape (circular annular shape in the present embodiment) so as to surround the slit 5a1 of the valve body 5a. According to the above configuration, since a support force from the rib 4a against the elastic valve body 5 can be favorably obtained via the annular protruding portion 5a2, the tight contact between the elastic valve body 5 and the elastic opening/closing portion 7 can be further improved. Note that the protruding portion 5a2 is not limited to the annular shape, and may be intermittently provided so as to surround the slit 5a1 of the valve body 5a.

The protruding portion 5a2 has a first step portion 5a3 provided in an annular shape so as to surround the slit 5a1 of the valve body 5a, and a second step portion 5a4 protruding from an outer peripheral edge of the first step portion 5a3 toward the axially distal side, the slit 5a1 of the valve body 5a has a linear shape when viewed in the axial direction, and the second step portion 5a4 consists of a pair of projecting portions 5a5 facing each other in a direction along the slit 5a1 of the valve body 5a.

The rib 4a is provided in an annular shape (circular annular shape in the present embodiment) so as to surround the connection port P. According to the above configuration, since a support force for the elastic valve body 5 can be favorably obtained by the annular rib 4a, the tight contact between the elastic valve body 5 and the elastic opening/closing portion 7 can be further improved. Note that the rib 4a is not limited to the annular shape, and may be intermittently provided so as to surround the connection port P.

The fixing portion 5b of the elastic valve body 5 includes a clamped portion 5b1 continuous over the entire periphery of the outer peripheral surface of the disk-shaped valve body 5a, a distal-side annular protruding portion 5b2 protruding over the entire periphery from the radially outer peripheral edge of the clamped portion 5b1 to the axially distal side, and a proximal-side annular protruding portion 5b3 protruding over the entire periphery from the radially outer peripheral edge of the clamped portion 5b1 to the axially proximal side. The connector body 4 includes a clamping portion 4b that clamps the clamped portion 5b1 of the fixing portion 5b of the elastic valve body 5.

The elastic valve body 5 of the female connector 2 is formed of, for example, rubber or an elastomer. The connector body 4 is formed of, for example, a resin.

The movable body restriction portion 10a is caught on the female connector 2 to restrict the movement of the female connector 2 toward the axially distal side relative to the elastic opening/closing portion 7. That is, the movable body restriction portion 10a has a movable body catching portion 10a1 that is caught on a movable body caught portion 11 of the female connector 2. The movable body restriction portion 10a can release the catch on the female connector 2 by swinging radially outward through restoration from elastic deformation. The movable body restriction portion 10a has an arm portion 10a2 extending radially outward and toward the axially distal side, and a protrusion 10a3 protruding radially inward from the end of the arm portion 10a2 on the axially distal side and constituting the movable body catching portion 10a1.

The movable body 10 includes a holding portion 10b that forms the holding opening Q and holds the elastic opening/closing portion 7, and a movable body base portion 10c having a cylindrical shape, for example, which is continuous with a root portion of the movable body restriction portion 10a located on the axially proximal side relative to the holding portion 10b. The holding portion 10b, the movable body restriction portion 10a, and the movable body base portion 10c form an integrated movable body 10. The movable body 10 has a first movable body restriction portion 10a and a second movable body restriction portion 10a, and the holding portion 10b is located between the first movable body restriction portion 10a and the second movable body restriction portion 10a, or the first movable body restriction portion 10a and the second movable body restriction portion 10a are provided so as to face each other (offset by 180°).

The housing restriction portion 9a is caught on a housing caught portion 12 to restrict the movement of the female connector 2 toward the axially distal side relative to the elastic opening/closing portion 7. That is, the housing restriction portion 9a has a housing catching portion 9a1 that is caught on the housing caught portion 12. The housing restriction portion 9a is caught on the housing caught portion 12 by swinging. The housing restriction portion 9a is caught on the housing caught portion 12 by swinging radially inward toward the flow passage tubular member 6 through restoration from elastic deformation.

The housing caught portion 12 is provided in the female connector 2. Note that the housing caught portion 12 may be provided in another member (for example, the movable body 10 having the movable body restriction portion 10a) that moves integrally with the female connector 2 during the connecting operation.

The elastic opening/closing portion 7 and the biasing portion 8 form an integrated elastic member. The elastic opening/closing portion 7 and the biasing portion 8 are formed of, for example, rubber or an elastomer. The movable body 10, the flow passage tubular member 6, and the housing 9 are formed of, for example, a resin.

The housing 9 has a housing base portion 9b that is continuous with the flow passage tubular member 6 and forms a hollow portion S in which the flow passage tubular member 6 is located, the housing restriction portion 9a that has the housing catching portion 9a1 on the axially distal side and is swingably continuous with the housing base portion 9b on the axially proximal side, and an operating portion 9c that extends from the housing restriction portion 9a toward the axially proximal side. The housing restriction portion 9a can release the catch on the housing caught portion 12 by swinging radially outward from the flow passage tubular member 6 due to elastic deformation, in response to a radially inward operation on the operating portion 9c.

The housing 9 has a first housing restriction portion 9a, a first operating portion 9c extending from the first housing restriction portion 9a to the axially proximal side, a second housing restriction portion 9a, and a second operating portion 9c extending from the second housing restriction portion 9a to the axially proximal side. The flow passage tubular member 6 is located between the first housing restriction portion 9a and the second housing restriction portion 9a, or the first housing restriction portion 9a and the second housing restriction portion 9a are provided so as to face each other (offset by 180°).

The flow passage tubular member 6 and the root portion of the housing base portion 9b form an integral member. The housing restriction portion 9a, the portion other than the root portion of the housing base portion 9b, and the operating portion 9c form an integral member.

Each of the male connector 3 and the female connector 2 is configured as a medical device. The male connector 3 and the female connector 2 can constitute, for example, an infusion set. The infusion set includes, for example, the male connector 3, the female connector 2, an infusion container, a drip chamber, and a medical tube. The fluid is not particularly limited, and is, for example, a medical fluid (such as an infusion agent, a nutrient, or an anticancer agent), a saline solution, or the like.

The embodiments of the present disclosure have been described above. However, the present disclosure is not limited to the above-described embodiments, and thus the above-described embodiments can be variously modified without departing from the gist of the present disclosure.

### Reference Signs List

- 1: Connection structure
- 2: Female connector
- 3: Male connector
- 4: Connector body
- 4a: Rib
- 4b: Clamping portion
- 5: Elastic valve body
- 5a: Valve body
- 5a1: Slit
- 5a2: Protruding portion
- 5a3: First step portion
- 5a4: Second step portion
- 5a5: Projecting portion
- 5b: Fixing portion
- 5b1: Clamped portion
- 5b2: Distal-side annular protruding portion
- 5b3: Proximal-side annular protruding portion
- 5c: Bottom surface
- 5d: Top surface
- 6: Flow passage tubular member
- 6a: Opening
- 7: Elastic opening/closing portion
- 7a: Inserted portion
- 7b: Slit
- 7c: Distal surface
- 8: Biasing portion
- 9: Housing
- 9a: Housing restriction portion
- 9a1: Housing catching portion
- 9b: Housing base portion
- 9c: Operating portion
- 10: Movable body
- 10a: Movable body restriction portion
- 10a1: Movable body catching portion
- 10a2: Arm portion
- 10a3: Protrusion
- 10b: Holding portion
- 10c: Movable body base portion
- 11: Movable body caught portion
- 12: Housing caught portion
- C1: Male connector flow passage
- C2: Female connector flow passage
- P: Connection port
- Q: Holding opening
- S: Hollow portion

## Claims

1. A connection structure comprising: a female connector; and a male connector connectable to the female connector,
wherein the female connector includes a connector body having a connection port and an elastic valve body,
the elastic valve body includes a valve body that closes the connection port and a fixing portion that extends from an outer peripheral surface of the valve body and is fixed to the connector body,
the male connector includes a flow passage tubular member that defines an axial direction and has an opening at a portion on an axially distal side, an elastic opening/closing portion, a biasing portion capable of biasing the elastic opening/closing portion toward the axially distal side, and a housing continuous with the flow passage tubular member,
the male connector changes from a first form in which the opening of the flow passage tubular member is closed by the elastic opening/closing portion to a second form in which the opening of the flow passage tubular member is opened by a connecting operation where the elastic valve body is pressed against a distal surface of the elastic opening/closing portion against a biasing force of the biasing portion to cause the elastic opening/closing portion to move toward an axially proximal side relative to the flow passage tubular member, such that the portion on the axially distal side of the flow passage tubular member protrudes from the elastic opening/closing portion toward the axially distal side,
the housing has a housing restriction portion that restricts movement of the female connector toward the axially distal side relative to the elastic opening/closing portion in accordance with a change from the first form to the second form,
the valve body has a slit penetrating the valve body and a protruding portion provided on a bottom surface so as to surround the slit,
the elastic opening/closing portion has an inserted portion inserted into the connection port by the connecting operation and a slit penetrating the elastic opening/closing portion through the inserted portion, and
the connector body has a rib against which the protruding portion is pressed as the valve body is pressed toward a deep side of the connection port by the inserted portion by the connecting operation.

2. The connection structure according to claim 1, wherein the inserted portion has an outer diameter equal to or less than an inner diameter of the connection port of the connector body.

3. The connection structure according to claim 1, wherein the flow passage tubular member defines a radial direction,
the male connector includes a movable body having a holding opening in which the elastic opening/closing portion is disposed and held and a movable body restriction portion that restricts the movement of the female connector toward the axially distal side relative to the elastic opening/closing portion by moving radially inward by a reaction force from the housing generated in accordance with the connecting operation, and
the inserted portion of the elastic opening/closing portion protrudes from the holding opening toward the axially distal side.

4. The connection structure according to claim 1, wherein the protruding portion is provided in an annular shape so as to surround the slit of the valve body.

5. The connection structure according to claim 1, wherein the rib is provided in an annular shape so as to surround the connection port.

6. The connection structure according to claim 1, wherein each of the male connector and the female connector is configured as a medical device.
